# FASCICULE DE BREVET EUROPEEN

(11) **EP 1 176 148 B1**
(45) Date de publication et mention de la délivrance du brevet: **03.09.2003**
(21) Numéro de dépôt: 01401839.4
(22) Date de dépôt: 10.07.2001
(51) Int. Cl.: C07D 513/04, A61K 31/542, A61P 25/00

(54) **Nouveaux dérivés de benzothiadiazine, leur procédé de préparation et les compositions pharmaceutiques qui les contiennent**
Benzothiadiazinderivate, Verfahren zu ihrer Herstellung und sie enthaltende Arzneimittel
Benzothiadiazine derivatives, processes for their preparation and pharmaceutical compositions containing them

(30) Priorité: 28.07.2000 FR 0009916
(43) Date de publication de la demande: 30.01.2002
(73) Titulaire: LES LABORATOIRES SERVIER, 92200 Neuilly sur Seine (FR)
(72) Inventeur: Cordi, Alex, 92150 Suresnes (FR); Desos, Patrice, 92400 Courbevoie (FR); Lefoulon, François, 45000 Orleans (FR); Lestage, Pierre, 78170 La Celle Saint Cloud (FR)

(56) Documents cités:
- WO-A-99/42456

## Description

La présente invention concerne de nouveaux dérivés de benzothiadiazine, leur procédé de préparation et les compositions pharmaceutiques qui les contiennent.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral reviews, 1992, 16, 13-24 ; Progress in Neurobiology, 1992, 39, 517-545).

Par ailleurs, d'innombrables travaux ont démontré durant les dernières années l'existence de sous-types réceptoriels aux aminoacides excitateurs et de leurs interactions fonctionnelles (Molecular Neuropharmacology, 1992, 2, 15-31).

Parmi ces récepteurs, le récepteur à l'AMPA ("a-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits très récemment comme modulant positivement les récepteurs AMPA des cellules neuronales (Journal of Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Enfin, le brevet EP 692 484 décrit un dérivé de benzothiadiazine possédant une activité facilitatrice sur le courant AMPA et la demande de brevet WO 99/42456 décrit entre autres certains dérivés de benzothiadiazine en tant que modulateurs des récepteurs AMPA.

Les dérivés de benzothiadiazine, objets de la présente invention, outre le fait qu'ils soient nouveaux, présentent, de manière surprenante, des activités pharmacologiques sur le courant AMPA nettement supérieures à celles des composés de structures proches décrits dans l'Art Antérieur. Ils sont utiles en tant que modulateurs AMPA pour le traitement ou la prévention des désordres mnémocognitifs associés à l'âge, aux syndromes anxieux ou dépressifs, aux maladies neurogénératives progressives, à la maladie d'Alzheimer, à la maladie de Pick, à la chorée d'Huntington, à la schizophrénie, aux séquelles des maladies neurodégénératives aiguës, aux séquelles de l'ischémie et aux séquelles de l'épilepsie.

Plus spécifiquement, la présente invention concerne les composés de formule **(I)** : 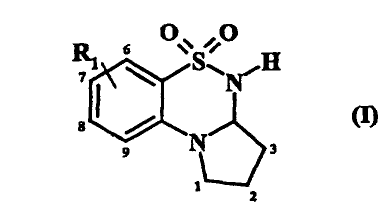 dans laquelle :
- **R**_{**1**}: représente un groupement hydroxy ou RCO-O-,
- **R**: représentent un groupement alkyle (C₁-C₆) linéaire ou ramifié éventuellement substitué par un groupement aryle, alkényle (C₂-C₆) linéaire ou ramifié éventuellement substitué par un groupement aryle , perhalogénoalkyle (C₁-C₆) linéaire ou ramifié, cycloalkyle (C₃-C₇), adamantyle, aryle ou hétéroaryle,
leurs isomères ainsi que leurs sels d'addition à un acide ou à une base pharmaceutiquement acceptable,
étant entendu que :
◆ R₁ est situé en position 7,
◆ par groupement aryle, on comprend groupement aromatique monocyclique ou un groupement bicyclique dans lequel au moins un des cycles est aromatique, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié) ou phényle (éventuellement substitué par un ou plusieurs groupements, identiques ou différents, halogène, alkyle (C₁-C₆) linéaire ou ramifié, perhalogénoalkyle (C₁-C₆) linéaire ou ramifié, hydroxy ou alkoxy (C₁-C₆) linéaire ou ramifié),
◆ par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène et soufre, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié) ou aminosulfonyle (éventuellement substitué par un ou plusieurs groupements alkyle (C₁-C₆) linéaire ou ramifié).

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

Les groupements aryles préférés sont les groupements phényle, naphtyle et tétrahydronaphtyle éventuellement substitués.

Les groupements hétéroaryles préférés sont les groupements pyridinyle, pyrrolyle, thiényle, furyle, imidazolyle, indolyle éventuellement substitués et de manière encore plus préférentielle les groupements pyridinyle, thiényle et furyle.

Certains composés préférés de l'invention sont les composés de formule (I) dans laquelle R₁ représente un groupement hydroxy.

D'autres composés préférés de l'invention sont les composés de formule (I) dans laquelle R₁ représente un groupement RCO-O.
Parmi les composés de l'invention, lorsque R₁ représente un groupement RCO-O, le groupement R est préférentiellement un groupement cycloalkyle (C₃-C₇), un groupement aryle ou un groupement hétéroaryle.

Les composés préférés de l'invention sont :
- le 5,5-dioxo-2,3,3a,4-tétrahydro-1*H*-pyrrolo[2,1-c][1,2,4]benzothiadiazin-7-ol
- le 5,5-dioxo-2,3,3a,4-tétrahydro-1*H*-pyrrolo[2,1-c][1,2,4]benzothiadiazin-7-yl benzoate
- le 5,5-dioxo-2,3,3a,4-tétrahydro-1*H*-pyrrolo[2,1-c][1,2,4]benzothiadiazin-7-yl-cyclohexane carboxylate
- le 5,5-dioxo-2,3,3a,4-tétrahydro-1*H*-pyrrolo[2,1-c][1,2,4]benzothiadiazin-7-yl-cyclobutane carboxylate
- le 5,5-dioxo-2,3,3a,4-tétrahydro-1*H*-pyrrolo[2,1-c][1,2,4] benzothiadiazin-7-yl-4-méthylbenzoate
- le 5,5-dioxo-2,3,3a,4-tétrahydro-1*H*-pyrrolo[2,1-c][1,2,4] benzothiadiazin-7-yl-3-thiophène carboxylate
- le 5,5-dioxo-2,3,3a,4-tétrahydro-1*H*-pyrrolo[2,1-c][1,2,4] benzothiadiazin-7-yl-2-thiophène carboxylate
- le 5,5-dioxo-2,3,3a,4-tétrahydro-1*H*-pyrrolo[2,1-c][1,2,4]benzothiadiazin-7-yl-3-furane carboxylate
- le 5,5-dioxo-2,3,3a,4-tétrahydro-1*H*-pyrrolo[2,1-c][1,2,4]benzothiadiazin-7-yl-2-furane carboxylate
- le 5,5-dioxo-2,3,3a,4-tétrahydro-1*H*-pyrrolo[2,1-c][1,2,4] benzothiadiazin-7-yl-nicotinate

L'invention s'étend également au procédé de préparation des composés de formule **(I)** caractérisé en ce que l'on utilise comme produit un composé de formule **(II)** : 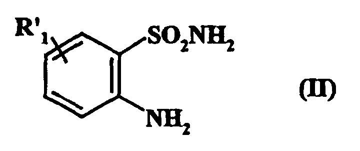 dans laquelle :
- **R'**_{**1**}: représente un groupement alkoxy (C₁-C₆) linéaire ou ramifié,
que l'on fait réagir avec le chlorure d'acide de formule **(III)** en présence d'une base, en milieu tétrahydrofurane ou acétonitrile :

**Cl - (CH**_{**2**}**)**_{**3**} **- COCl** (III)

pour conduire au composé de formule **(IV)** : 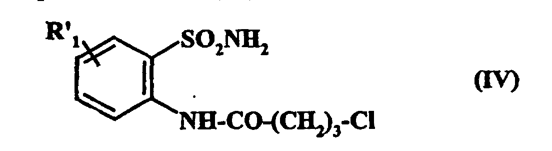 dans laquelle R'₁ est tel que défini précédemment,
qui est alors cyclisé en milieu basique, pour conduire au composé de formule **(V)** : 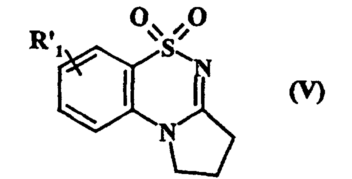 dans laquelle R'₁ est tel que défini précédemment,
qui subit une réduction, en milieu alcoolique ou diméthylformamide, en présence de borohydrure de sodium, pour conduire au composé de formule **(VI)** : 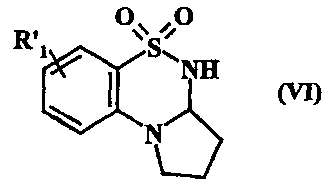 dans laquelle R'₁ est tel que défini précédemment,
composé de formule (VI) qui
subit l'action du tribromure de bore
pour conduire
au composé de formule **(I/a),** cas particulier des composés de formule **(I)** : 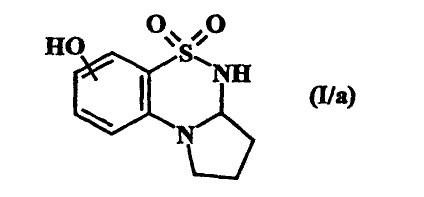 composé de formule (I/a) dont on peut acyler la fonction hydroxy pour conduire aux composés (I/d) tels que le groupement hydroxy du noyau phényle a été transformé en groupement R-CO-O dans lequel R est tel que défini dans la formule (I),
composés (I/a) et (I/d) qui constituent l'ensemble des composés de formule (I),
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

L'invention s'étend aussi aux compositions pharmaceutiques renfermant comme principe actif un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, les suspensions buvables, etc...

La posologie utile est adaptable selon la nature et la sévérité de l'affection, la voie d'administration ainsi que selon l'âge et le poids du patient. Cette posologie varie de 1 à 500 mg par jour en une ou plusieurs prises.

Les exemples suivants illustrent l'invention mais ne la limitent en aucune façon.

Les produits de départ utilisés sont des produits connus ou préparés selon des modes opératoires connus.

Les structures des composés décrits dans les exemples ont été déterminées selon les techniques spectrophotométriques usuelles (infrarouge, RMN, spectrométrie de masse, ...).

### EXEMPLE 1 : 5,5-Dioxo-2,3,3a,4-tétrahydro-1H-pyrrolo[2,1-c][1,2,4]benzothiadiazin-7-ol

### Stade A : N-[2-(Aminosulfonyl)-4-méthoxyphényl]-4-chlorobutanamide

A une solution contenant 96,4 mmoles de 2-amino-5-méthoxybenzènesulfonamide dans 200 ml de tétrahydrofurane (THF), on ajoute 144 mmoles de triéthylamine puis goutte à goutte une solution contenant 135 mmoles du chlorure de l'acide 4-chlorobutanoïque dans 30 ml de THF. Après une nuit d'agitation à température ambiante, le THF est évaporé et le résidu est repris par de l'eau. Après extraction à l'acétate d'éthyle, la phase organique est lavée, séchée. Après évaporation, on obtient le produit attendu sous forme d'huile.

### Stade B: 5,5-Dioxo-7-méthoxy-2,3-dihydro-1H-pyrrolo[2,1-c] [1,2,4]benzothiadiazine

Le produit obtenu au stade précédent est agité une nuit, à température ambiante, dans 320 ml d'une solution aqueuse de soude 1N. Après addition de 50 ml d'acétate d'éthyle et agitation vigoureuse, le produit attendu qui précipite est filtré, rincé et séché.

| *Microanalyse élémentaire :* | | | | |
|---|---|---|---|---|
| | *C%* | *H%* | *N%* | *S%* |
| *calculé* | *52,37* | *4,79* | *11,10* | *12,71* |
| *trouvé* | *52,30* | *4,79* | *10,98* | *12,96* |

### Stade C : 5,5-Dioxo-7-méthoxy-2,3,3a,4-tétrahydro-1H-pyrrolo[2,1-c][1,2,4] benzothiadiazine

A une suspension contenant 35,5 mmoles du produit obtenu au stade précédent dans 40 ml de diméthylformamide (DMF) sont ajoutés 106,5 mmoles de borohydrure de sodium. Après une nuit d'agitation à température ambiante, le milieu réactionnel est refroidi puis 150 ml d'une solution glacée d'acide chlorhydrique 1N sont ajoutés au mélange précédent. Le produit attendu précipite et est filtré.
*Point de fusion : 193-198°C*

| *Microanalyse élémentaire :* | | | | |
|---|---|---|---|---|
| | *C%* | *H%* | *N%* | *S%* |
| *calculé* | *51,95* | *5,55* | *11,02* | *12,61* |
| *trouvé* | *51,60* | *5,59* | *10,87* | *12,69* |

### Stade D : 5,5-Dioxo-2,3,3a,4-tétrahydro-1H-pyrrolo[2,1-c][1,2,4]benzothiadiazin-7-ol

A une suspension contenant 26,7 mmoles du produit obtenu au stade précédent dans 350ml de dichlorométhane maintenue à -60°C sous azote sont ajoutés goutte à goutte 79,3 mmoles de tribromure de bore. La température est maintenue une heure puis l'cnsemble revient à température ambiante et est agité une nuit. Après refroidissement du milieu réactionnel dans un bain de glace, 100 ml d'eau sont ajoutés et le système biphasique qui se forme est agité vigoureusement. La suspension ainsi formée est filtrée.

Le solide blanc obtenu est lavé à l'eau, à l'éther, séché et conduit au produit attendu.
*Point de fusion* : *237-242°C*

| *Microanalyse élémentaire :* | | | | |
|---|---|---|---|---|
| | *C%* | *H%* | *N%* | *S%* |
| *calculé* | *49,99* | *5,03* | *11,66* | *13,34* |
| *trouvé* | *49,82* | *5,17* | *11,44* | *13,64* |

### EXEMPLE 7 : 5,5-Dioxo-2,3,3a,4-tétrahydro-1H-pyrrolo[2,1-c][1,2,4]benzothiadiazin-7-yl acétate

A une suspension contenant 4,16 mmoles du composé décrit dans l'exemple 1 dans 30 ml de dichlorométhane, sont ajoutés 60 mg de 4-diméthylaminopyridine et 4,16 mmoles d'anhydride acétique. Après 20 minutes d'agitation, le milieu réactionnel est dilué avec 30 ml de dichlorométhane. La phase organique est lavée, séchée puis évaporée. Le produit attendu est obtenu après reprise du solide blanc formé dans de l'éther isopropylique et filtration.
*Point de fusion : 163-165°C*

| *Microanalyse élémentaire :* | | | | |
|---|---|---|---|---|
| | *C%* | *H%* | *N%* | *S%* |
| *calculé* | *51,05* | *5,00* | *9,92* | *11,36* |
| *trouvé* | *51,21* | *5,06* | *9,73* | *11,43* |

### EXEMPLE 8 : 5,5-Dioxo-2,3,3a,4-tétrahydro-1H-pyrrolo[2,1-c][1,2,4]benzothiadiazin-7-yl acétate, isomère α dextrogyre

### EXEMPLE 9 : 5,5-Dioxo-2,3,3a,4-tétrahydro-1H-pyrrolo[2,1-c][1,2,4]benzothiadiazin-7-yl acétate, isomère β lévogyre

Les isomères α et β du composé décrit dans l'exemple 7 sont séparés par chromatographie chirale sur colonne Chiralpak AD® en utilisant comme solvant d'élution un mélange n-heptane/éthanol/triéthylamine (450/550/2). Après séparation, chaque isomère est purifié par chromatographie sur colonne de silice en utilisant comme solvant d'élution un mélange dichlorométhane/méthanol/triéthylamine (950/50/1).

### EXEMPLE 8 :

[α]^{D}₂₀ = + 191,1° (C = 5 mg/ml éthanol 95 %)

| *Microanalyse élémentaire :* | | | | |
|---|---|---|---|---|
| | *C%* | *H%* | *N%* | *S%* |
| *calculé* | *51,05* | *5,00* | *9,92* | *11,36* |
| *trouvé* | *51,07* | *4,96* | *9,71* | *11,57* |

### EXEMPLE 9 :

[α]^{D}₂₀ = - 192,8° (C = 5 mg/ml éthanol 95 %)

| *Microanalyse élémentaire :* | | | | |
|---|---|---|---|---|
| | *C%* | *H%* | *N%* | *S%* |
| *calculé* | *51,05* | *5,00* | *9,92* | *11,36* |
| *trouvé* | *51,30* | *4,98* | *9,76* | *11,09* |

### EXEMPLE 10 : 5,5-Dioxo-2,3,3a,4-tétrahydro-1H-pyrrolo[2,1-c][1,2,4]benzothiadiazin-7-yl pivalate

1,66 mmoles du composés de l'exemple 1 dans 40 ml d'acétonitrile sont agités une nuit à température ambiante en présence de 3,33 mmoles de chlorométhylpivalate et d'une quantité catalytique de dicyclohexyl-18-crown-6. La suspension est filtrée et le filtrat évaporé à sec. Le résidu est repris dans du dichlorométhane et cette phase organique est lavée par une solution d'acide chlorhydrique 1N puis par une solution aqueuse saturée de chlorure de sodium. Après séchage et évaporation, le résidu huileux obtenu est cristallisé dans un mélange éther/cyclohexane pour conduire au produit attendu.
*Point de fusion : 198-202°C*

| *Microanalyse élémentaire :* | | | | |
|---|---|---|---|---|
| | *C%* | *H%* | *N%* | *S%* |
| *calculé* | *55,54* | *6,21* | *8,64* | *9,88* |
| *trouvé* | *56,01* | *6,46* | *8,36* | *9,52* |

### EXEMPLE 11 : 5,5-Dioxo-2,3,3a,4-tétrahydro-1H-pyrrolo[2,1-c][1,2,4]benzothiadiazin-7-yl benzoate

Le produit attendu est obtenu selon le procédé décrit dans l'exemple 7 en remplaçant l'anhydride acétique par l'anhydride benzoïque.
*Point de fusion : 195°C*

| *Microanalyse élémentaire :* | | | | |
|---|---|---|---|---|
| | *C%* | *H%* | *N%* | *S%* |
| *calculé* | *59,29* | *4,68* | *8,13* | *9,31* |
| *trouvé* | *59,62* | *4,58* | *8,07* | *9,18* |

### EXEMPLE 11a : 5,5-Dioxo-2,3,3a,4-tétrahydro-1H-pyrrolo[2,1-c][1,2,4]benzothiadiazin-7-yl benzoate, isomère α dextrogyre

### EXEMPLE 11a :5,5-Dioxo-2,3,3a,4-tétrahydro-1H-pyrrolo[2,1-c][1,2,4]benzothiadiazin-7-yl benzoate, isomère β lévogyre

Les isomères α et ***β*** du composé décrit dans l'exemple 11 sont séparés par chromatographie chirale sur colonne Whelk® 01 en utilisant comme solvant d'élution l'isopropanol. Après séparation, chaque isomère est purifié par chromatographie sur colonne de silice en utilisant comme éluant un mélange dichlorométhane/méthanol (99/1)

### EXEMPLE 11a :

[α]^{D}₂₀= +151,6° (C = 5mg/ml DMSO)

| *Microanalyse élémentaire :* | | | | |
|---|---|---|---|---|
| | *C%* | *H%* | *N%* | *S%* |
| *calculé* | *59,29* | *4,68* | *8,13* | *9,31* |
| *trouvé* | *59,07* | *4,69* | *8,01* | *9,16* |

### EXEMPLE 12 : 5,5-Dioxo-2,3,3a,4-tétrahydro-1H-pyrrolo[2,1-c][1,2,4]benzothiadiazin-7-yl-4-chlorobenzoate

Le produit attendu est obtenu selon le procédé décrit dans l'exemple 7 en remplaçant l'anhydride acétique par le chlorure de l'acide p.chlorobenzoïque et en ajoutant 1,1 équivalents de triéthylamine.
*Point de fusion : 160°C*

| *Microanalyse élémentaire :* | | | | | |
|---|---|---|---|---|---|
| | *C%* | *H%* | *N%* | *S%* | *Cl%* |
| *calculé* | *53,90* | *3,99* | *7,39* | *8,46* | *9,36* |
| *trouvé* | *53,83* | *3,95* | *7,29* | *8,57* | *9,50* |

Les composés décrits dans les exemples suivants ont été préparés par condensation du composé de l'exemple 1 avec le chlorure d'acide correspondant en présence de 1,5 équivalents de triéthylamine et d'une quantité catalytique de diméthylaminopyridine.

### EXEMPLE 14 : 5,5-Dioxo-2,3,3a,4-tétrahydro-1H-pyrrolo[2,1-c][1,2,4] benzothiadiazin-7-yl-cyclohexane carboxylate

*Point de fusion :* 157 °C

| *Microanalyse élémentaire* | | | | |
|---|---|---|---|---|
| | C% | H% | N% | S% |
| calculé | 58,27 | 6,33 | 7,99 | 9,15 |
| trouvé | 58,77 | 6,47 | 8,04 | 8,93 |

### EXEMPLE 15 : 5,5-Dioxo-2,3,3a,4-tétrahydro-1H-pyrrolo[2,1-c][1,2,4] benzothiadiazin-7-yl-adamantane carboxylate

*Point de fusion :* 199-203 °C

| *Microanalyse élémentaire* | | | | |
|---|---|---|---|---|
| | C% | H% | N% | S% |
| calculé | 62,66 | 6,51 | 6,96 | 7,97 |
| trouvé | 62,93 | 6,62 | 6,9 | 7,79 |

### EXEMPLE 16 : 5,5-Dioxo-2,3,3a,4-tétrahydro-1H-pyrrolo[2,1-c][1,2,4] benzothiadiazin-7-yl-cyclopentane carboxylate

*Point de fusion* : 148-150 °C

| *Microanalyse élémentaire* | | | | |
|---|---|---|---|---|
| | C% | H% | N% | S% |
| calculé | 57,13 | 5,99 | 8,33 | 9,53 |
| trouvé | 57,41 | 6,02 | 8,21 | 9,11 |

### EXEMPLE 17 : 5,5-Dioxo-2,3,3a,4-tétrahydro-1H-pyrrolo[2,1-c][1,2,4] benzothiadiazin-7-yl-cyclobutane carboxylate

*Point de fusion* : 166-170 °C

| *Microanalyse élémentaire* | | | | |
|---|---|---|---|---|
| | C% | H% | N% | S% |
| calculé | 55,89 | 5,63 | 8,69 | 9,95 |
| trouvé | 55,97 | 5,7 | 8,54 | 9,92 |

### EXEMPLE 18 : 5,5-Dioxo-2,3,3a,4-tétrahydro-1H-pyrrolo[2,1-c][1,2,4] benzothiadiazin-7-yl-cyclopropane carboxylate

*Point de fusion* : 169-171 °C

| *Microanalyse élémentaire* | | | | |
|---|---|---|---|---|
| | C% | H% | N% | S% |
| calculé | 54,53 | 5,23 | 9,08 | 10,4 |
| trouvé | 54,58 | 5,3 | 8,7 | 10,38 |

### EXEMPLE 19 : 5,5-Dioxo-2,3,3a,4-tétrahydro-1H-pyrrolo[2,1-c][1,2,4] benzothiadiazin-7-yl-1-naphtalène carboxylate

*Point de fusion* : 248-251 °C

| *Microanalyse élémentaire* | | | | |
|---|---|---|---|---|
| | C% | H% | N% | S% |
| calculé | 63,95 | 4,60 | 7,10 | 8,13 |
| trouvé | 63,69 | 4,54 | 7,03 | 7,91 |

### EXEMPLE 20 : 5,5-Dioxo-2,3,3a,4-tétrahydro-1H-pyrrolo[2,1-c][1,2,4] benzothiadiazin-7-yl-2-naphtalène carboxylate

*Point de fusion* : 207-210 °C

| *Microanalyse élémentaire* | | | | |
|---|---|---|---|---|
| | C% | H% | N% | S% |
| calculé | 63,95 | 4,60 | 7,10 | 8,13 |
| trouvé | 64,22 | 4,70 | 7,15 | 7,73 |

### EXEMPLE 21 : 5,5-Dioxo-2,3,3a,4-tétrahydro-1H-pyrrolo[2,1-c][1,2,4] benzothiadiazin-7-yl-[1,1'-biphényl]-4-carboxylate

*Point de fusion* : 249-253 °C

| *Microanalyse élémentaire* | | | | |
|---|---|---|---|---|
| | C% | H% | N% | S% |
| calculé | 65,70 | 4,79 | 6,66 | 7,63 |
| trouvé | 65,36 | 4,75 | 6,57 | 7,50 |

### EXEMPLE 22 : 5,5-Dioxo-2,3,3a,4-tétrahydro-1H-pyrrolo[2,1-c][1,2,4] benzothiadiazin-7-yl-phénylacétate

*Point de fusion* : 169-171 °C

| *Microanalyse élémentaire* | | | | |
|---|---|---|---|---|
| | C% | H% | N% | S% |
| calculé | 60,32 | 5,06 | 7,82 | 8,95 |
| trouvé | 60,56 | 5,00 | 7,54 | 9,16 |

### EXEMPLE 23 : 5,5-Dioxo-2,3,3a,4-tétrahydro-1H-pyrrolo[2,1-c][1,2,4]-benzothiadiazin-7-yl-3-phényl-2-propénoate

*Point de fusion* : 193-198 °C

| *Microanalyse élémentaire* | | | | |
|---|---|---|---|---|
| | C% | H% | N% | S% |
| calculé | 61,61 | 4,9 | 7,56 | 8,66 |
| trouvé | 61,81 | 5,00 | 7,21 | 8,56 |

### EXEMPLE 24 : 5,5-Dioxo-2,3,3a,4-tétrahydro-1H-pyrrolo[2,1-c][1,2,4] benzothiadiazin-7-yl-4-méthoxybenzoate

*Point de fusion* : 216-221 °C

| *Microanalyse élémentaire* | | | | |
|---|---|---|---|---|
| | C% | H% | N% | S% |
| calculé | 57,74 | 4,85 | 7,48 | 8,56 |
| trouvé | 57,05 | 4,77 | 7,39 | 8,48 |

### EXEMPLE 25 : 5,5-Dioxo-2,3,3a,4-tétrahydro-1H-pyrrolo[2,1-c][1,2,4] benzothiadiazin-7-yl-(4-diméthylamino) benzoate

*Point de fusion* : 232-235 °C

| *Microanalyse élémentaire* | | | | |
|---|---|---|---|---|
| | C% | H% | N% | S% |
| calculé | 58,9 | 5,46 | 10,85 | 8,28 |
| trouvé | 58,83 | 5,48 | 10,76 | 8,43 |

### EXEMPLE 26 : 5,5-Dioxo-2,3,3a,4-tétrahydro-1H-pyrrolo[2,1-c][1,2,4] benzothiadiazin-7-yl-3-chlorobenzoate

*Point de fusion* : 243-247 °C

| *Microanalyse élémentaire* | | | | | |
|---|---|---|---|---|---|
| | C% | H% | N% | S% | Cl% |
| calculé | 53,9 | 3,99 | 7,39 | 8,46 | 9,36 |
| trouvé | 53,78 | 4,03 | 7,23 | 8,23 | 9,89 |

### EXEMPLE 27 : 5,5-Dioxo-2,3,3a,4-tétrahydro-1H-pyrrolo[2,1-c][1,2,4] benzothiadiazin-7-yl-4-cyanobenzoate

*Point de fusion* : 260-264 °C

| *Microanalyse élémentaire* | | | | |
|---|---|---|---|---|
| | C% | H% | N% | S% |
| calculé | 58,53 | 4,09 | 11,38 | 8,68 |
| trouvé | 58,90 | 4,16 | 11,42 | 8,71 |

### EXEMPLE 28 : 5,5-Dioxo-2,3,3a,4-tétrahydro-1H-pyrrolo[2,1-c][1,2,4] benzothiadiazin-7-yl-4-méthylbenzoate

*Point de fusion* : 198-200 °C

| *Microanalyse élémentaire* | | | | |
|---|---|---|---|---|
| | C% | H% | N% | S% |
| calculé | 60,32 | 5,06 | 7,82 | 8,95 |
| trouvé | 60,36 | 5,09 | 7,67 | 8,57 |

### EXEMPLE 29 : 5,5-Dioxo-2,3,3a,4-tétrahydro-1H-pyrrolo[2,1-c][1,2,4] benzothiadiazin-7-yl-3-méthylbenzoate

*Point de fusion* : 214-218 °C

| *Microanalyse élémentaire* | | | | |
|---|---|---|---|---|
| | C% | H% | N% | S% |
| calculé | 60,32 | 5,06 | 7,82 | 8,95 |
| trouvé | 60,04 | 5,04 | 7,68 | 8,64 |

### EXEMPLE 30 : 5,5-Dioxo-2,3,3a,4-tétrahydro-1H-pyrrolo[2,1-c][1,2,4] benzothiadiazin-7-yl-2-méthylbenzoate

*Point de fusion* : 218-221 °C

| *Microanalyse élémentaire* | | | | |
|---|---|---|---|---|
| | C% | H% | N% | S% |
| calculé | 60,32 | 5,06 | 7,82 | 8,95 |
| trouvé | 60,25 | 5,03 | 7,65 | 8,61 |

### EXEMPLE 31 : 5,5-Dioxo-2,3,3a,4-tétrahydro-1H-pyrrolo[2,1-c][1,2,4] benzothiadiazin-7-yl-3-cyanobenzoate

*Point de fusion* : 203-206 °C

| *Microanalyse élémentaire* | | | | |
|---|---|---|---|---|
| | C% | H% | N% | S% |
| calculé | 58,53 | 4,09 | 11,38 | 8,68 |
| trouvé | 58,50 | 4,16 | 11,17 | 8,35 |

### EXEMPLE 32 : 5,5-Dioxo-2,3,3a,4-tétrahydro-1H-pyrrolo[2,1-c][1,2,4] benzothiadiazin-7-yl-pentafluorobenzoate

*Point de fusion* : 205-209 °C

| *Microanalyse élémentaire* | | | | |
|---|---|---|---|---|
| | C% | H% | N% | S% |
| calculé | 47,01 | 2,55 | 6,45 | 7,38 |
| trouvé | 46,95 | 2,56 | 6,33 | 7,05 |

### EXEMPLE 33 : 5,5-Dioxo-2,3,3a,4-tétrahydro-1H-pyrrolo[2,1-c][1,2,4] benzothiadiazin-7-yl-3-thiophène carboxylate

*Point de fusion :* 208-212 °C

| *Microanalyse élémentaire* | | | | |
|---|---|---|---|---|
| | C% | H% | N% | S% |
| calculé | 51,42 | 4,03 | 7,99 | 18,30 |
| trouvé | 51,68 | 4,01 | 8,07 | 17,84 |

### EXEMPLE 34 : 5,5-Dioxo-2,3,3a,4-tétrahydro-1H-pyrrolo[2,1-c][1,2,4] benzothiadiazin-7-yl-2-thiophène carboxylate

*Point de fusion* : 212-214 °C

| *Microanalyse élémentaire* | | | | |
|---|---|---|---|---|
| | C% | H% | N% | S% |
| calculé | 51,42 | 4,03 | 7,99 | 18,3 |
| trouvé | 51,33 | 4,43 | 8,03 | 18,48 |

### EXEMPLE 35 : 5,5-Dioxo-2,3,3a,4-tétrahydro-1H-pyrrolo[2,1-c][1,2,4] benzothiadiazin-7-yl-3-furane carboxylate

*Point de fusion* : 185-187 °C

| *Microanalyse élémentaire* | | | | |
|---|---|---|---|---|
| | C% | H% | N% | S% |
| calculé | 53,89 | 4,22 | 8,38 | 9,59 |
| trouvé | 53,89 | 4,22 | 8,36 | 9,52 |

### EXEMPLE 36 : 5,5-Dioxo-2,3,3a,4-tétrahydro-1H-pyrrolo[2,1-c][1,2,4] benzothiadiazin-7-yl-2-furane carboxylate

*Point de fusion* : 205-208 °C

| *Microanalyse élémentaire* | | | | |
|---|---|---|---|---|
| | C% | H% | N% | S% |
| calculé | 53,89 | 4,22 | 8,38 | 9,59 |
| trouvé | 53,55 | 4,23 | 8,16 | 9,59 |

### EXEMPLE 37 : 5,5-Dioxo-2,3,3a,4-tétrahydro-1H-pyrrolo[2,1-c][1,2,4] benzothiadiazin-7-yl-nicotinate

*Point de fusion* : 227-230 °C

| *Microanalyse élémentaire* | | | | |
|---|---|---|---|---|
| | C% | H% | N% | S% |
| calculé | 55,64 | 4,38 | 12,17 | 9,28 |
| trouvé | 55,32 | 4,43 | 11,63 | 9,43 |

### EXEMPLE 38 : 5,5-Dioxo-2,3,3a,4-tétrahydro-1H-pyrrolo[2,1-c][1,2,4] benzothiadiazin-7-yl-4-pyridine carboxylate, chlorhydrate

*Point de fusion* : 243-247 °C

| *Microanalyse élémentaire* | | | | | |
|---|---|---|---|---|---|
| | C% | H% | N% | S% | Cl% |
| calculé | 50,33 | 4,22 | 11,00 | 8,4 | 9,28 |
| trouvé | 50,00 | 4,54 | 10,69 | 8,18 | 8,91 |

### EXEMPLE 39 : 5,5-Dioxo-2,3,3a,4-tétrahydro-1H-pyrrolo[2,1-c][1,2,4] benzothiadiazin-7-yl-2-pyridine carboxylate, chlorhydrate

*Point de fusion* : 227-230 °C

| *Microanalyse élémentaire* | | | | | |
|---|---|---|---|---|---|
| | C% | H% | N% | S% | Cl% |
| calculé | 50,33 | 4,22 | 11,00 | 8,4 | 9,28 |
| trouvé | 50,93 | 4,30 | 10,85 | 8,36 | 7,21 |

### ETUDE PHARMACOLOGIQUE DES PRODUITS DE L'INVENTION

### Etude des courants excitateurs induit par l'AMPA dans les oocytes de Xenopus

### a - Méthode :

Des ARNm sont préparés à partir de cortex cérébral de rat mâle Wistar par la méthode au guanidium thiocyanate/phénol/chloro forme. Les ARNm Poly (A⁺) sont isolés par chromatographie sur oligo-dT cellulose et injectés à raison de 50 ng par oocyte. Les oocytes sont laissés 2 à 3 jours en incubation à 18°C pour permettre l'expression des récepteurs puis stockés à 8-10°C.

L'enregistrement électrophysiologique est réalisé dans une chambre en plexiglass® à 20-24°C en milieu OR2 (J. Exp. Zool., 1973, 184, 321-334) par la méthode du "voltage-clamp" à 2 électrodes, une 3ème électrode placée dans le bain servant de référence.

Tous les composés sont appliqués via le milieu d'incubation et le courant électrique est mesuré à la fin de la période d'application. L'AMPA est utilisé à la concentration de 10 µM. Pour chaque composé étudié, on définit la concentration doublant (EC2X) ou quintuplant (EC5X) l'intensité du courant induit par l'AMPA seul (5 à 50 nA).

### b - Résultats :

Les composés de l'invention potentialisent très fortement les effets excitateurs de l'AMPA et leur activité est très nettement supérieure à celles des composés de référence.

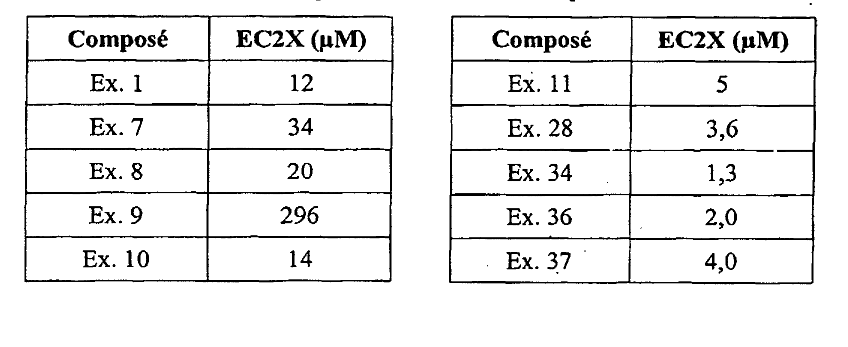

### COMPOSITION PHARMACEUTIQUE

Formule de préparation pour 1000 comprimés dosés à 100 mg

| | |
|---|---|
| Composé de l'exemple 1 | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule **(I)** : 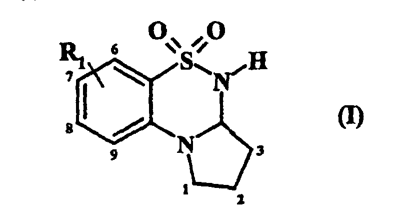 dans laquelle :
**R**_{**1**} représente un groupement hydroxy ou RCO-O-,
**R** représente un groupement alkyle (C₁-C₆) linéaire ou ramifié éventuellement substitué par un groupement aryle, alkényle (C₂-C₆) linéaire ou ramifié éventuellement substitué par un groupement aryle , perhalogénoalkyle (C₁-C₆) linéaire ou ramifié, cycloalkyle (C₃-C₇), adamantyle, aryle ou hétéroaryle,
leurs isomères ainsi que leurs sels d'addition à un acide ou à une base pharmaceutiquement acceptable,
étant entendu que :
◆ R₁ est situé en position 7,
◆ par groupement aryle, on comprend groupement aromatique monocyclique ou un groupement bicyclique dans lequel au moins un des cycles est aromatique, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié), aminosulfonyle (éventuellement substitué par un ou plusieurs groupements alkyle (C₁-C₆) linéaire ou ramifié) ou phényle (éventuellement substitué par un ou plusieurs groupements, identiques ou différents, halogène, alkyle (C₁-C₆) linéaire ou ramifié, perhalogénoalkyle (C₁-C₆) linéaire ou ramifié, hydroxy ou alkoxy (C₁-C₆) linéaire ou ramifié),
◆ par groupement hétéroaryle, on comprend groupement aromatique monocyclique ou un groupement bicyclique dans lequel au moins un des cycles est aromatique, contenant un, deux ou trois hétéroatomes, identiques ou différents, choisis parmi azote, oxygène et soufre, éventuellement substitué par un ou plusieurs groupements, identiques ou différents, halogène, alkyle (C₁-C₆) linéaire ou ramifié, alkoxy (C₁-C₆) linéaire ou ramifié, perhalogénoalkyle (C₁-C₆) linéaire ou ramifié, perhalogénoalkoxy (C₁-C₆) linéaire ou ramifié, hydroxy, cyano, nitro, amino (éventuellement substitué par un ou plusieurs groupements alkyle (C₁-C₆) linéaire ou ramifié) ou aminosulfonyle (éventuellement substitué par un ou plusieurs groupements alkyle (C₁-C₆) linéaire ou ramifié).

2. Composés de formule (I) selon la revendication 1 **caractérisés en ce que** R₁ représente un groupement hydroxy.

3. Composés de formule (I) selon la revendication 1 **caractérisés en ce que** R₁ représente un groupement RCO-O-.

4. Composés de formule (I) selon la revendication 3 **caractérisés en ce que** R représente un groupement cycloalkyle (C₃-C₇), un groupement aryle ou un groupement hétéroaryle.

5. Composés de formule (I) selon la revendication 1 qui sont :
- le 5,5-dioxo-2,3,3a,4-tétrahydro-1*H*-pyrrolo[2,1-c][1,2,4] benzothiadiazin-7-ol
- le 5,5-Dioxo-2,3,3a,4-tétrahydro-1*H*-pyrrolo[2,1-c][1,2,4] benzothiadiazin-7-yl benzoate
- le 5,5-dioxo-2,3,3a,4-tétrahydro-1*H*-pyrrolo[2,1-c][1,2,4] benzothiadiazin-7-yl cyclohexane carboxylate
- le 5,5-dioxo-2,3,3a,4-tétrahydro-1*H*-pyrrolo[2,1-c][1,2,4]benzothiadiazin-7-yl-cyclobutane carboxylate
- le 5,5-dioxo-2,3,3a,4-tétrahydro-1*H*-pyrrolo[2,1-c][1,2,4]benzothiadiazin-7-yl-4-méthylbenzoate
- le 5,5-dioxo-2,3,3a,4-tétrahydro-1*H*-pyrrolo[2,1-c][1,2,4]benzothiadiazin-7-yl-3-thiophène carboxylate
- le 5,5-dioxo-2,3,3a,4-tétrahydro-1*H*-pyrrolo[2,1-c][1,2,4]benzothiadiazin-7-yl-2-thiophène carboxylate
- le 5,5-dioxo-2,3,3a,4-tétrahydro-1*H*-pyrrolo[2,1-c][1,2,4]benzothiadiazin-7-yl-3-furane carboxylate
- le 5,5-dioxo-2,3,3a,4-tétrahydro-1*H*-pyrrolo[2,1-c][1,2,4] benzothiadiazin-7-yl-2-furane carboxylate
- le 5,5-dioxo-2,3,3a,4-tétrahydro-1*H*-pyrrolo[2,1-c][1,2,4]benzothiadiazin-7-yl-nicotinate.

6. Procédé de préparation des composés de formule (I) selon la revendication 1 **caractérisé en ce que** l'on utilise comme produit un composé de formule **(II)** : 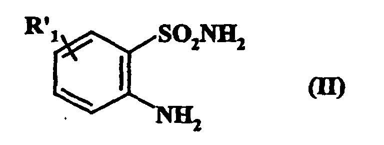 dans laquelle :
**R'**_{**1**} représente un groupement alkoxy (C₁-C₆) linéaire ou ramifié,
que l'on fait réagir avec le chlorure d'acide de formule (III) en présence d'une base, en milieu tétrahydrofurane ou acétonitrile :
**Cl-(CH**_{**2**}**)**_{**3**}**-COCl** (III)
pour conduire au composé de formule (IV) : 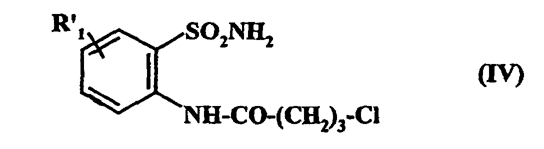 dans laquelle R'₁ est tel que défini précédemment,
qui est alors cyclisé en milieu basique, pour conduire au composé de formule **(V)** : 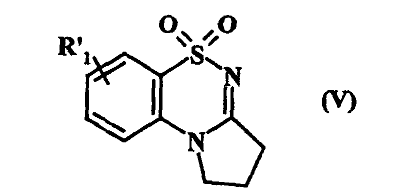 dans laquelle R'₁ est tel que défini précédemment,
qui subit une réduction, en milieu alcoolique ou diméthylformamide, en présence de borohydrure de sodium, pour conduire au composé de formule (VI) : 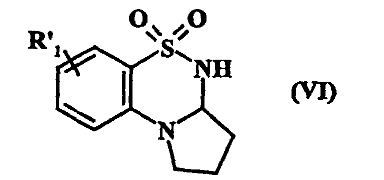 dans laquelle R'₁ est tel que défini précédemment,
composé de formule (VI) qui
subit l'action du tribromure de bore
pour conduire
au composé de formule (I/a), cas particulier des composés de formule (I) : 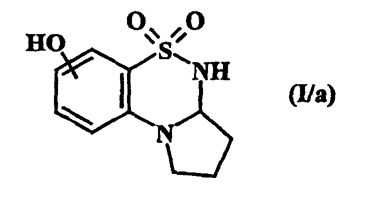 composé de formule (I/a) dont on peut acyler la fonction hydroxy pour conduire aux composés (I/d) tels que le groupement hydroxy du noyau phényle a été transformé en groupement R-CO-O dans lequel R est tel que défini dans la formule (I),
composés (I/a) et (I/d) qui constituent l'ensemble des composés de formule (I),
que l'on purifie le cas échéant selon une technique classique de purification, dont on sépare éventuellement les isomères selon une technique classique de séparation et que l'on transforme, si on le souhaite, en leurs sels d'addition à un acide ou à une base pharmaceutiquement acceptable.

7. Compositions pharmaceutiques contenant comme principe actif un composé selon l'une quelconque des revendications 1 à 5 en combinaison avec un ou plusieurs excipients ou véhicules inertes, non toxiques, pharmaceutiquement acceptables.

8. Compositions pharmaceutiques selon la revendication 7 contenant comme principe actif un composé selon l'une quelconque des revendications 1 à 5 utiles en tant que médicaments, comme modulateurs AMPA.

## Patentansprüche

1. Verbindungen der Formel **(I)**: 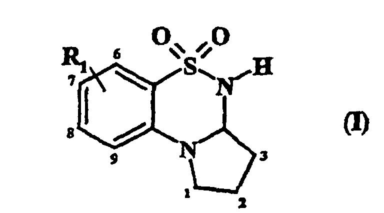 in der:
**R**_{**1**} eine Hydroxygruppe oder eine Gruppe RCO-O-,
**R** eine geradkettige oder verzweigte, gegebenenfalls durch eine Arylgruppe substituierte geradkettige oder verzweigte (C₁-C₆)-Alkylgruppe, geradkettige oder verzweigte, gegebenenfalls durch eine Arylgruppe substituierte (C₂-C₆)-Alkenylgruppe, geradkettige oder verzweigte (C₁-C₆)-Perhalogenalkylgruppe, (C₃-C₇)-Cycloalkylgruppe, Adamantylgruppe, Arylgruppe oder Heteroarylgruppe bedeuten,
deren Isomere sowie deren Additionssalze mit einer pharmazeutisch annehmbaren Säure oder Base,
mit der Maßgabe, daß:
◆ R₁ in der 7-Stellung steht,
◆ unter einer Arylgruppe, eine monocyclische oder bicyclische aromatische Gruppe zu verstehen ist, bei der mindestens einer der Ringe aromatisch ist, und die gegebenenfalls durch ein oder mehrere gleichartige oder verschiedenartige Halogenatome, geradkettige oder verzweigte (C₁-C₆)-Alkylgruppen, geradkettige oder verzweigte (C₁-C₆)-Alkoxygruppen, geradkettige oder verzweigte (C₁-C₆)-Perhalogenalkylgruppen, geradkettige oder verzweigte (C₁-C₆)-Perhalogenalkoxygruppen, Hydroxygruppen, Cyanogruppen, Nitrogruppen, Aminogruppen (die gegebenenfalls durch eine oder mehrere geradkettige oder verzweigte (C₁-C₆)-Alkylgruppen substituiert sind), Aminosulfonylgruppen (die gegebenenfalls durch eine oder mehrere geradkettige oder verzweigte (C₁-C₆)-Alkylgruppen substituiert sind) oder Phenylgruppen (die gegebenenfalls substituiert sind durch ein oder mehrere gleichartige oder verschiedene Halogenatome, geradkettige oder verzweigte (C₁-C₆)-Alkylgruppen, geradkettige oder verzweigte (C₁-C₆)-Perhalogenalkylgruppen, Hydroxygruppen oder geradkettige oder verzweigte (C₁-C₆)-Alkoxygruppen) substituiert ist,
◆ unter einer Heteroarylgruppe eine monocyclische oder bicyclische aromatische Gruppe zu verstehen ist, bei der mindestens einer der Ringe aromatisch ist und ein, zwei oder drei gleichartige oder verschiedenartige Heteroatome ausgewählt aus Stickstoff, Sauerstoff und Schwefel enthält und gegebenenfalls substituiert ist durch ein oder mehrere gleichartige oder verschiedene Halogenatome, geradkettige oder verzweigte (C₁-C₆)-Alkylgruppen, geradkettige oder verzweigte (C₁-C₆)-Alkoxygruppen, geradkettige oder verzweigte (C₁-C₆)-Perhalogenalkylgruppen, geradkettige oder verzweigte (C₁-C₆)-Perhalogenalkoxygruppen, Hydroxygruppen, Cyanogruppen, Nitrogruppen, Aminogruppen (die gegebenenfalls durch eine oder mehrere geradkettige oder verzweigte (C₁-C₆)-Alkylgruppen substituiert sind) oder Aminosulfonylgruppen (die gegebenenfalls durch eine oder mehrere geradkettige oder verzweigte (C₁-C₆)-Alkylgruppen substituiert sind).

2. Verbindungen der Formel (I) nach Anspruch 1, **dadurch gekennzeichnet, daß** R₁ eine Hydroxygruppe bedeutet.

3. Verbindungen der Formel (I) nach Anspruch 1, **dadurch gekennzeichnet, daß** R₁ eine Gruppe RCO-O- bedeutet.

4. Verbindungen der Formel (I) nach Anspruch 3, **dadurch gekennzeichnet, daß** R eine (C₃-C₇)-Cycloalkylgruppe, eine Arylgruppe oder eine Heteroarylgruppe bedeutet.

5. Verbindungen der Formel (I) nach Anspruch 1, nämlich:
- 5,5-Dioxo-2,3,3a,4-tetrahydro-1*H*-pyrrolo[2,1-c][1,2,4]benzothiadiazin-7-ol,
- 5,5-Dioxo-2,3,3a,4-tetrahydro-1*H*-pyrrolo[2,1-c][1,2,4]benzothiadiazin-7-yl-benzoat,
- 5,5-Dioxo-2,3,3a,4-tetrahydro-1*H*-pyrrolo[2,1-c][1,2,4]benzothiadiazin-7-yl-cyclohexancarboxylat,
- 5,5-Dioxo-2,3,3a,4-tetrahydro-1*H*-pyrrolo[2,1-c][1,2,4]benzothiadiazin-7-yl-cyclobutancarboxylat,
- 5,5-Dioxo-2,3,3a,4-tetrahydro-1*H*-pyrrolo[2,1-c][1,2,4]benzothiadiazin-7-yl-4-methylbenzoat,
- 5,5-Dioxo-2,3,3a,4-tetrahydro-1*H*-pyrrolo[2,1-c][1,2,4]benzothiadiazin-7-yl-3-thiophencarboxylat,
- 5,5-Dioxo-2,3,3a,4-tetrahydro-1*H*-pyrrolo[2,1-c][1,2,4]benzothiadiazin-7-yl-2-thiophencarboxylat,
- 5,5-Dioxo-2,3,3a,4-tetrahydro-1*H*-pyrrolo[2,1-c][1,2,4]benzothiadiazin-7-yl-3-furancarboxylat,
- 5,5-Dioxo-2,3,3a,4-tetrahydro-1*H*-pyrrolo[2,1-c][1,2,4]benzothiadiazin-7-yl-2-furancarboxylat,
- 5,5-Dioxo-2,3,3a,4-tetrahydro-1*H*-pyrrolo[2,1-c][1,2,4]benzothiadiazin-7-yl-nicotinat.

6. Verfahren zur Herstellung der Verbindungen der Formel (I) nach Anspruch 1, **dadurch gekennzeichnet, daß** man als Ausgangsprodukt eine Verbindung der Formel (II) verwendet: 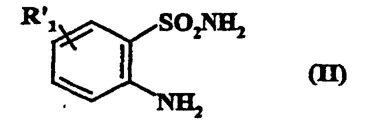 in der:
**R'**_{**1**} eine geradkettige oder verzweigte (C₁-C₆)-Alkoxygruppe bedeutet,
welche man mit dem Säurechlorid der Formel (III) in Gegenwart einer Base und in einem Tetrahydrofuran- oder Acetonitril-Medium umsetzt:
**Cl - (CH**_{**2**}**)**_{**3**} **- COCl** (III)
zur Bildung der Verbindung der Formel (IV): 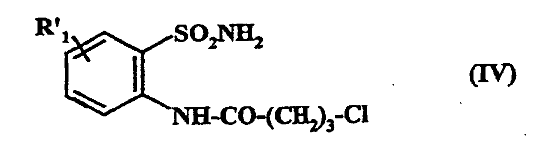 in der R'₁ die oben angegebenen Bedeutungen besitzt,
welche man anschließend in basischem Medium cyclisiert zur Bildung der Verbindung der Formel (V): 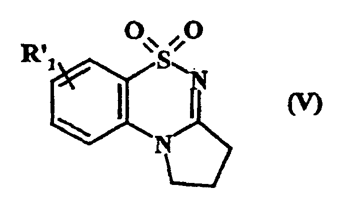 in der R'₁ die oben angegebenen Bedeutungen besitzt,
welche man in alkoholischem oder Dimethylformamid-Medium in Gegenwart von Natriumborhydrid reduziert zur Bildung der Verbindung der Formel (VI): 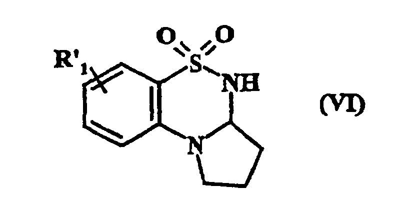 in der R'₁ die oben angegebenen Bedeutungen besitzt,
welche Verbindung der Formel (VI) man der Einwirkung von Bortribromid unterzieht
zur Bildung
der Verbindung der Formel (I/a), einem Sonderfall der Verbindungen der Formel (I): 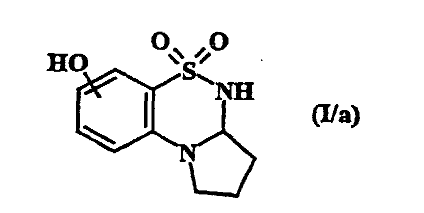 bei welcher Verbindung der Formel (I/a) man die Hydroxyfunktion acylieren kann zur Bildung der Verbindungen (I/d), derart, daß die Hydroxygruppe des Phenylkerns in eine Gruppe R-CO-O umgewandelt wird, in der R die bezüglich der Formel (I) angegebenen Bedeutungen besitzt,
welche Verbindungen (I/a) und (I/d), welche die Gesamtheit der Verbindungen der Formel (I) bilden, man gegebenenfalls mit Hilfe einer klassischen Reinigungsmethode reinigt, man gegebenenfalls mit Hilfe einer klassischen Trennungsmethode in ihre Isomeren auftrennt und gewünschtenfalls in ihre Additionssalze mit einer pharmazeutisch annehmbaren Säure oder Base umwandelt.

7. Pharmazeutische Zubereitungen enthaltend als Wirkstoff eine Verbindung nach einem der Ansprüche 1 bis 5 in Kombination mit einem oder mehreren inerten, nichttoxischen, pharmazeutisch annehmbaren Trägermaterialien oder Bindemitteln.

8. Pharmazeutische Zubereitungen nach Anspruch 7 enthaltend als Wirkstoff eine Verbindung nach einem der Ansprüche 1 bis 5 nützlich als Arzneimittel, wie AMPA-Modulatoren.

## Claims

1. Compounds of formula **(I)** : 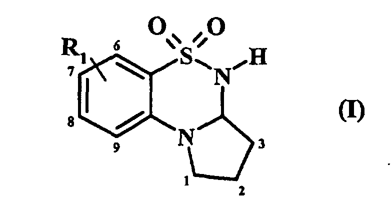 wherein :
**R**_{**1**} represents a hydroxy or RCO-O- group,
**R** represents a linear or branched (C₁-C₆)alkyl group optionally substituted by an aryl group, a linear or branched (C₂-C₆)alkenyl group optionally substituted by an aryl group, a linear or branched (C₁-C₆)perhaloalkyl group, a (C₃-C₇)cycloalkyl group, an adamantyl group, an aryl group or a heteroaryl group,
their isomers and addition salts thereof with a pharmaceutically acceptable acid or base, it being understood that:
◆ R₂ is located in the 7-position,
◆ "aryl group" is understood to mean a monocyclic aromatic group or a bicyclic group in which at least one of the rings is aromatic, optionally substituted by one or more identical or different groups: halogen, linear or branched (C₁-C₆)alkyl, linear or branched (C₁-C₆)alkoxy, linear or branched (C₁-C₆)perhaloalkyl, linear or branched (C₁-C₆)perhaloalkoxy, hydroxy, cyano, nitro, amino (optionally substituted by one or more linear or branched (C₁-C₆)alkyl groups), aminosulphonyl (optionally substituted by one or more linear or branched (C₁-C₆)alkyl groups) or phenyl (optionally substituted by one or more identical or different groups: halogen, linear or branched (C₁-C₆)alkyl, linear or branched (C₁-C₆)perhaloalkyl, hydroxy or linear or branched (C₁-C₆)alkoxy),
◆ "heteroaryl group" is understood to mean a monocyclic aromatic group or a bicyclic group in which at least one of the rings is aromatic, containing one, two or three identical or different hetero atoms selected from nitrogen, oxygen and sulphur, optionally substituted by one or more identical or different groups: halogen, linear or branched (C₁-C₆)alkyl, linear or branched (C₁-C₆)alkoxy, linear or branched (C₁-C₆)-perhaloalkyl, linear or branched (C₁-C₆)perhaloalkoxy, hydroxy, cyano, nitro, amino (optionally substituted by one or more linear or branched (C₁-C₆)alkyl groups) or aminosulphonyl (optionally substituted by one or more linear or branched (C₁-C₆)alkyl groups).

2. Compounds of formula (I) according to claim 1, **characterised in that** R₁ represents a hydroxy group.

3. Compounds of formula (I) according to claim 1, **characterised in that** R₁ represents an RCO-O- group.

4. Compounds of formula (I) according to claim 3, **characterised in that** R represents a (C₃-C₇)cycloalkyl group, an aryl group or a heteroaryl group.

5. Compounds of formula (I) according to claim 1 which are :
- 5,5-dioxo-2,3,3a,4-tetrahydro-1*H*-pyrrolo[2,1-c][1,2,4]benzothiadiazin-7-ol
- 5,5-dioxo-2,3,3a,4-tetrahydro-1*H*-pyrrolo[2,1-c][1,2,4]benzothiadiazin-7-yl benzoate
- 5,5-dioxo-2,3,3a,4-tetrahydro-1*H*-pyrrolo[2,1-c][1,2,4]benzothiadiazin-7-yl cyclohexanecarboxylate
- 5,5-dioxo-2,3,3a,4-tetrahydro-1*H*-pyrrolo[2,1-c][1,2,4]benzothiadiazin-7-yl cyclobutanecarboxylate
- 5,5-dioxo-2,3,3a,4-tetrahydro-1*H*-pyrrolo[2,1-c][1,2,4]benzothiadiazin-7-yl 4-methylbenzoate
- 5,5-dioxo-2,3,3a,4-tetrahydro-1*H*-pyrrolo[2,1-c][1,2,4]benzothiadiazin-7-yl 3-thiophenecarboxylate
- 5,5-dioxo-2,3,3a,4-tetrahydro-1*H*-pyrrolo[2,1-c][1,2,4]benzothiadiazin-7-yl 2-thiophenecarboxylate
- 5,5-dioxo-2,3,3a,4-tetrahydro-1*H*-pyrrolo[2,1-c][1,2,4]benzothiadiazin-7-yl 3-furan-carboxylate
- 5,5-dioxo-2,3,3a,4-tetrahydro-1*H*-pyrrolo[2,1-c][1,2,4]benzothiadiazin-7-yl 2-furan-carboxylate
- 5,5-dioxo-2,3,3a,4-tetrahydro-1*H*-pyrrolo[2,1-c][1,2,4]benzothiadiazin-7-yl nicotinate.

6. Process for the preparation of compounds of formula (I) according to claim 1, **characterised in that** there is used as starting material a compound of formula (II) : 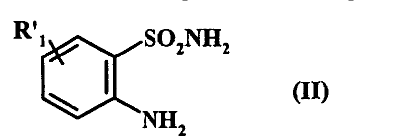 wherein:
**R'**_{**1**} represents a linear or branched (C₁-C₆)alkoxy group,
which is reacted with the acid chloride of formula (III) in the presence of a base, in a tetrahydrofuran or acetonitrile medium:
**Cl - (CH**_{**2**}**)**_{**3**} **- COCl** (III)
to yield a compound of formula (IV) : 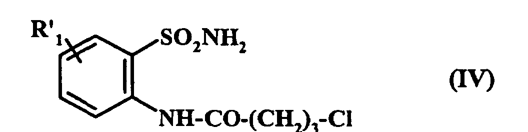 wherein R'₁ is as defined hereinbefore,
which is then cyclised in a basic medium, to yield a compound of formula (V) : 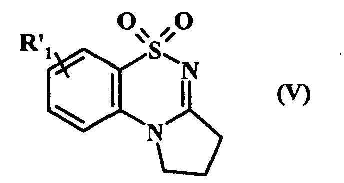 wherein R'₁ is as defined hereinbefore,
which is subjected to reduction, in an alcoholic medium or in a dimethylformamide medium, in the presence of sodium borohydride, to yield a compound of formula (VI) : 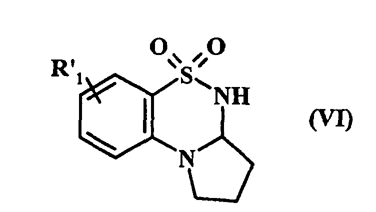 wherein R'₁ is as defined hereinbefore,
which compound of formula (VI) :
is subjected to the action of boron tribromide,
to yield:
the compound of formula (I/a), a particular case of the compounds of formula (I) : 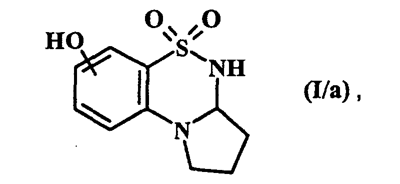
the hydroxy function of which compound of formula (I/a) may be acylated to yield the compounds (I/d) wherein the hydroxy group of the phenyl nucleus has been converted to an R-CO-O group wherein R is as defined for formula (I),
which compounds (I/a) and (I/d) constitute the totality of the compounds of formula (I),
which are purified, if necessary, according to a conventional purification technique, are separated, where appropriate, into their isomers according to a conventional separation technique, and converted, if desired, into addition salts with a pharmaceutically acceptable acid or base.

7. Pharmaceutical compositions comprising as active ingredient a compound according to any one of claims 1 to 5 in combination with one or more inert, non-toxic, pharmaceutically acceptable excipients or carriers.

8. Pharmaceutical compositions according to claim 7 comprising as active ingredient a compound according to any one of claims 1 to 5 for use as medicaments, as AMPA modulators.
